# EUROPEAN PATENT APPLICATION

(11) **EP 4 582 986 A1**
(43) Date of publication of application: **09.07.2025**
(21) Application number: 24156779.1
(22) Date of filing: 09.02.2024
(51) Int. Cl.: G06F 21/62, G06F 21/64, G16H 10/60, G16H 40/20, H04L 9/32, H04L 9/00, H04L 9/40

(54) **COMPUTER IMPLEMENTED METHOD FOR OPERATING A DATA POOL IN A HEALTHCARE ENVIRONMENT**

(30) Priority: 05.01.2024 WO PCT/CN2024/070768
(71) Applicant: Koninklijke Philips N.V., 5656 AG Eindhoven (NL)
(72) Inventor: KELLY, Declan Patrick, Eindhoven (NL); HELAOUI, Rim, Eindhoven (NL)
(74) Representative: Philips Intellectual Property & Standards

(57) **Abstract**

Computer implemented method for operating a data pool in a healthcare environment, comprising: providing a distributed ledger with a plurality of data blocks (S10); wherein each of the data block comprises at least a cryptographic hash for a respective data set of the data pool and meta data describing the respective data set; providing an access to the respective data set by an access control method (S20).

## Description

### FIELD OF THE INVENTION

The invention relates to a computer implemented method for operating a data pool in a healthcare environment, to a device, to a computer program and to a computer readable medium.

### BACKGROUND OF THE INVENTION

Data in medical environment can have a big value for analysis for various interest groups. The data is generated in different locations over the world, for example hospitals, research institutions or universities. The operation of data pools managing different data sets from different providers is a major challenge regarding data quality and transparency.

It has now become apparent that there is a further need to provide a method for operating a data pool in a healthcare environment.

### SUMMARY OF THE INVENTION

In view of the above, it is an object of the present invention to provide a method that allows for improving the operating of a data pool in a healthcare environment.

These and other objects, which become apparent upon reading the following description, are solved by the subject matter of the independent claims. The invention provides a method, a device and a computer program. The dependent claims refer to preferred embodiments of the invention.

In one aspect of the present disclosure, a computer implemented method for operating a data pool in a healthcare environment is provided, comprising: providing a distributed ledger with a plurality of data blocks; wherein each of the data blocks comprises at least a cryptographic hash for a respective data set of the data pool and meta data describing the respective data set; providing an access to the respective data set by an access control method.

The term data pool, as used herein, is to be understood broadly and may relate to a collection of data sets from different sources. The different sources may comprise a hospital, a university and research institute.

The term operating a data pool, as used herein, is to be understood broadly and may relate to organizing and/or managing a data pool. Operating a data pool may comprise providing access to a data pool. Operating may comprise adding a new data set to the data pool.

The term healthcare environment, as used herein, is to be understood broadly and may relate to any entity which engages with health care issues. The healthcare environment may relate to a hospital, a university, an insurance company and/or a research institution.

The term distributed ledger, as used herein, is to be understood broadly and may relate to a decentralized and synchronized database that is maintained across multiple locations and/or participants. The distributed ledger may record information and a change of information regarding data sets in a secure manner. The distributed ledger may be based on blockchain technology. The distributed ledger may comprise a blockchain with a plurality of data blocks. The distributed ledger may comprise a smart contract used to execute one or more programs when certain conditions are met. This may be advantageous in order to automate process steps of the method described above and below. The blockchain may be a permissioned blockchain. The blockchain may be consortium blockchain. The blockchain may be federated blockchain. The network of the blockchain can be either joined as a miner, as a validator or as an observer. The blockchain can maintain tokens (like a cryptocurrency) that reflect the relative stake among the validators (i.e. those we can create/vote on adding blocks). Original creators of the blockchain who contribute the data sets tracked on the blockchain may distribute the total set of tokens in proportion to the value they create. Token ownership may be tracked and modified on the blockchain as with cryptocurrencies. Revenue from licensing the data may be distributed in proportion to the token ownership.

The term data block, as used herein, is to be understood broadly and may relate to a unit of information comprising a list of records. The records may be indicative of a data set from a medical environment. The records may comprise one or more of the following: a previous cryptographic hash, a timestamp, a version of the blockchain, a summary of the total data set, a price for sharing data (e.g. in form of tokens), a cryptographic hash of the respective data set. These data blocks are preferably linked together in a chronological and immutable chain. Each data block may include a cryptographic hash of the previous data block. This may create a secure and transparent ledger.

The term cryptographic hash, as used herein, is to be understood broadly and may relate to a unique identifier for the data set or the data block and in particular for the records of the data block. The cryptographic hash may be a mathematical function that takes the records of the data block and produces a fixed size string of characters. The cryptographic hash may allow to check whether the respective data set has been amended. In a blockchain, each data block may further comprise a cryptographic hash of the previous data block, forming a blockchain. This process may ensure the integrity of the data because altering any data block would require changing all subsequent data blocks making the blockchain resistant to tempering and providing a secure and transparent ledger for transactions.

The term data set, as used herein, is to be understood broadly and may relate to any data in the medical environment. The data set may comprise patient data, treatment data, medication data, research data and/or insurance data. The data set may further comprise data indicative of the origin of the data set, the legal status of the data set, the provider of the data set and/or the storage of the data set.

The term meta data as used herein is to be understood broadly and may relate to any data configured to describe a data set. The meta data may comprise one or more of the following: size of a data set, data set quality, source of a data set, time stamp of a data set.

The term access control method, as used herein, is to be understood broadly and may relate to any method for enabling a controlled access to a data set. The access control method may be one of the following public key encryption/ private key decryption, role-based access control, discretionary access control, mandatory access control, attribute-based access control, role-based access control. An access control method may comprise user names and corresponding passwords.

It has been found out that typically no institution has a complete data set sufficient to create models that can be applied across different demographics. This may create an incentive for institutions to pool data and then the pooled data can be used to create a valuable AI model (thereby allowing the institutions to generate value from their data). One problem may be that the quality of the data is important and, given the benefit of being part of such a consortium, there is an incentive to supply data even if the data is not high quality. Furthermore, users of the data (i.e. licensees) will need to be able to trace back the source of the data (e.g. in case of an audit) and show that the data was obtained legally and that the source of the data has the legal right to license the data for the use-case.

The basic idea of the invention proposes to use a blockchain to manage a data pool. The blockchain comprises data blocks indicative of the respective data sets in the data pool. These data blocks which comprise meta data and cryptographic hashes of the respective data sets. The data blocks enable a trustworthy management of data sets as any change in the blockchain and/or in the respective data sets can be determined. In this manner, also a quality of the respective data sets can be guaranteed. Furthermore, also the expansion of the data pool can be controlled in a transparent and safe way. A further advantage of the method is the possibility to store the data sets offline, for example in the respective sources such as hospital or research institutes. The access to the respective data sets is enabled by controlled access methods such public/private encryption. In general this may allow different interest groups to participate on data sets from different sources, wherein the quality of the data sets is guaranteed and the management of the data pool is transparent and safe. The data pool may serve as data base for federated learning. The data set contributors may get tokens for their data sets. The tokens may be monetized by revenue streams for licensing the data. The tokens may be sold to third parties, thereby monetizing future revenue streams from licensing the data. The blockchain can be queried for available data, for example, a third party can query (though any of the nodes in the blockchain peer-to-peer network) the total available data or for the availability of a target subset of data (i.e. they can define their requirements and see if the blockchain can support). This can be supported by smart contracts. The blockchain may have smart contracts defining the datasets and subsets that can be offered for what price and under what conditions.

According to an embodiment, the respective data set may be stored off the distributed ledger. The data set may be stored at a source, for example a hospital or a research institute. An authorized user of the distributed ledger may receive a link to the respective data set. This may increase the efficiency of the method as no additional storage capacities for the data sets are needed. The data sets may be encrypted by a public key and the user has corresponding private key to access the data set. The authorized user may be allowed to copy the data set from the source and to store it locally such that the complete data set is replicated.

According to an embodiment, the access control method may comprise key encryption. The term key encryption, as used herein is to be understood broadly and may relate to the public/private key encryption technology. The key encryption may relate to asymmetric key encryption. The data sets may be encrypted by a public key and an authorized user may have a corresponding private key to access/ to decrypt the encrypted data set. This may increase the security of the operating of the data pool.

According to an embodiment, the data block may comprise legal information. The legal information may comprise a legal basis for owning, sharing and/or selling the data set. The legal information may comprise corresponding conditions and regions/geographies. This may be advantageous for users of the data as it increases the reliability and transparency of the data set.

According to an embodiment, the method may further comprise adding a further data block to the distributed ledger. This may enable the expansion of the data pool. This may increase the applicability of the method. The further data block may preferably comprise the same the data structure as the previous data blocks. The adding of a further data block may comprise a proposing of a further data block and assessing of the proposed data block. The assessing may be based on a quality measure. A contributor may be anonymous or not anonymous. In case the contributor is not anonymous, the contributor may be pre-validated and then whitelisted to be able to propose adding a new data set. This may ensure traceability of the data combined. Furthermore, there could be a wider consortium of potential participants (e.g. all US hospitals that meet certain criteria) that are whitelisted and can propose to add data. Such a construction would support the evolution of high-quality data sets with clear traceability and explicit rules on the sharing of the value created. If the data block is added, then the contributor who proposed the data may receive tokens, either the price requested or a set price. The token value given to this contributor may either be newly created or taken from existing token holders in proportion to their current take.

According to an embodiment, the adding of a further data block may comprise comparing the meta data of the further data block with a quality measure and deriving a comparison result; and adding the further data block based on the comparison result to the distributed ledger. The comparing may comprise a comparing of the corresponding data set with a quality measure. The quality measure may be for example novelty of the data set. If the data set is comparable to existing data, then it may be deemed to not add value. This may advantageously increase the quality of the data sets managed by the method. The comparing may be executed by a smart contract stored in the distributed ledger. The term smart contract, as used herein, is to be understood broadly and may relate to a program configured to be executed when certain conditions are met. For example, when a new data set is proposed, the smart contract is executed. Smart Contracts could be used to request data and define, for data sets that meet the criteria, the payment that will be given. For example, the smart contract defines the data set requirements (e.g. demographics, size) and the license conditions (e.g. US only) needed to satisfy the contract and the payment for supplying such a data set.

According to an embodiment, the quality measure may comprise one or more of the following: data set size, data set quality, data set novelty, legal status of data set. This may advantageously increase the quality of the data sets managed by the method.

According to an embodiment, the comparing may be carried by a validator, wherein the validator may be one of the following: authority, provider of a data set. Another term for provider is contributor. This may advantageously increase the quality of the data sets managed by the method. For example, one validator is enough to assess whether a new data set is added to the distributed ledger.

According to an embodiment, the comparing may be carried by a plurality of validators, wherein the validator may be one of the following: authority, provider of a data set. This may advantageously increase the quality of the data sets managed by the method. In other words, a plurality of validators assesses whether a new data set is added to the distributed ledger. The right to vote can be given for example in one of two ways:
Proof-of-stake where institutions that have already added data sets to the distributed ledger gain the right to vote either equally or in proportion to the size/quality of their contribution or in proportion to their position in the distributed ledger. So founding institutes may retain control.

Proof-of-authority where certain institutes may have the right to vote. This could be perpetually limited to the founding members or could be extended to new institutes after successfully adding data sets.

According to an embodiment, the method may further comprise adding a licensing block to the distributed ledger, wherein the licensing block may comprise at least information on licensing conditions for the respective data set. The licensing block may also only count for a part of a respective data set. The licensing block may also be assessed by a validator in the same manner as a new data block. In other words, licensing of the data may also be recorded on the blockchain. A new licensing block will be added to indicate the licensing conditions and once the block is approved, the data will be released to the licensee. The licensing conditions can be checked against the legal release for all licensed data, this may mean that only a subset of the data is licensed in some cases. This may be advantageous as multiple institutions (e.g. hospitals) can pool their data in order to create a high value data set for either their own use (creating a shared AI model) or to license to others (e.g. health tech companies). Licensees may be ensured that they can trace back the data used and the legal basis - specifically they can show that the data set they have, corresponds to that in the blockchain (i.e. distributed ledger) (by comparing cryptographic hashes) and may show that the license they have to the data is covered by the legal release of the data contributor. Licensees may need to pay for the data. This can be done through traditional payment channels, however, it could also be done via crypto currencies. For example, the public key used on the data sharing blockchain could be used to transfer bitcoins to this agent on the bitcoin blockchain.

According to an embodiment, the method may further comprise updating the data block, wherein the updating may comprise a replacing of the respective data set with a new data set and replacing the respective data block with a new data block. In other words, a new data set replace may replace a previous data set. This may be recorded in the corresponding data bock in the distributed ledger. This may increase the applicability of the method for operating a data pool.

According to an embodiment, the distributed ledger may comprise a summary block, wherein the summary block may comprise a summary of all data sets and/or wherein the distributed ledger may comprise one or more tokens, wherein the tokens may be indicative of a stake on the data sets in the data pool.

In other words, the distributed ledger could maintain a summary block of the total set of data, the cumulative data set from combining all included data sets. This cumulative summary block may include summaries based on different licensing conditions. The summary block can be generated from the data blocks. The summary block may be part of the latest data block or may be a separate data block in the distributed ledger. This may increase the transparency of the data pool.

A further aspect relates to a device comprising means for carrying out the method described above.

A further aspect relates to a computer program comprising instructions which, when the program is executed by a computer, cause the computer to carry out the method described above.

A last aspect relates to a computer readable medium comprising instructions which, when executed by a computer, cause the computer to carry out the method described above.

The computer program might be stored on a computer unit, which might also be part of an embodiment. This computer unit may be configured to perform or induce performing of the steps of the method described above. Moreover, it may be configured to operate the components of the above-described device. The computing unit can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further, on, the computer program might be able to provide all necessary steps to fulfil the procedure of an exemplary embodiment of the method as described above. According to a further exemplary embodiment of the present invention, a computer readable medium, such as a CD-ROM, USB stick or the like, is presented wherein the computer readable medium has a computer program stored on it which computer program is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program available for downloading is provided, which computer program is arranged to perform a method according to one of the previously described embodiments of the invention.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 shows a schematic view of a method for operating a data pool in a healthcare environment;
Fig. 2 shows a schematic view of a blockchain of a distributed ledger;
Fig. 3 shows a schematic view of a part of the method;
Fig. 4 shows a schematic view of a part of the method; and
Fig. 5 shows a schematic view of a part of the method.

### DETAILED DESCRIPTION OF THE DRAWINGS

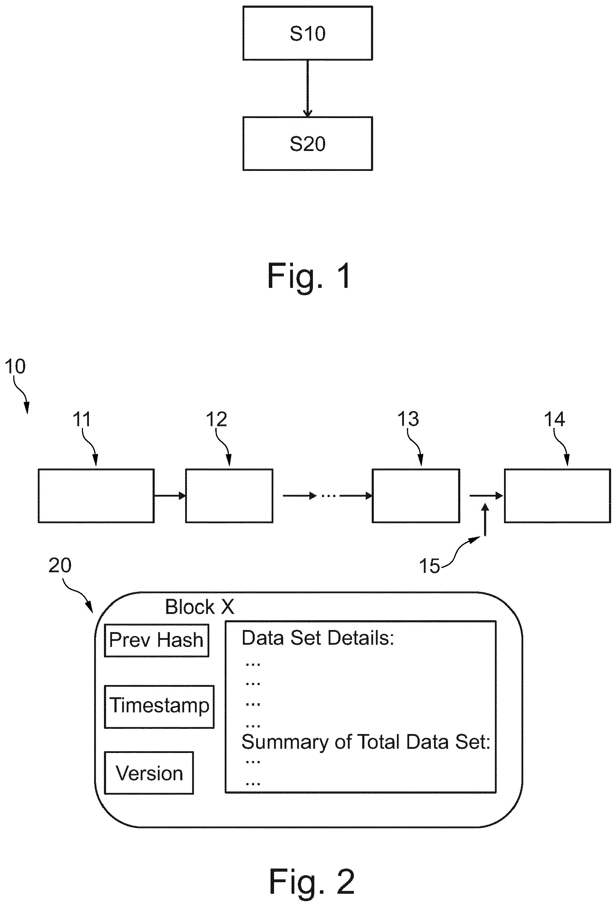

Fig. 1 shows a schematic view of a method for operating a data pool in a medical environment.

Step S10 comprises providing a distributed ledger with a plurality of data blocks. The distributed ledger uses in the present example a blockchain. The medical environment may comprise in the present example a hospital and a research Institute. The distributed ledger may comprise two data blocks, one of the hospital and one of the research Institute. The respective data blocks comprise in the present example a cryptographic hash for the respective data set of the data pool and meta data describing the respective data set. In the present example, the first status that is locally stored in the university and the second data set is locally stored in the research Institute. The distributed ledger is in this example, is stored decentral at a computer in the hospital and at a computer in the research institute. Alternatively, the distributed ledger may be hosted central by the hospital. The hospital may then be one of the founding members of the data pool.

Step S20 comprises providing access to the respective data set by an access control method. The access control method may be in the present example, a public key encryption/ private key decryption method. The hospital encrypts the first data set with a public key and provides a private key for decryption to the research Institute. The research Institute encrypts the first data set with this private key. The research Institute encrypts a second data set with a further public key and provides a corresponding private key for decryption to the hospital. The hospital decrypts the second data set with this private key.

The method may further comprise adding a further data block to the distributed ledger. The adding may comprise a comparing of meta data stored in the data block with a quality measure. The comparing may comprise a comparing the corresponding data set with a quality measure. The quality measure may be in the present example the data set quality. The data set quality may relate in the present example to the completeness of the data set. The meta data may be indicative of the completeness of the data set. The quality measure may comprise a threshold value for the completeness of the data set. The comparison may lead a completeness above or below the threshold. In case the completeness is above the threshold, the comparison is positive otherwise negative. In case of a positive comparison result the data block may be added to the distributed ledger otherwise not. The comparing may be carried one or more validators. In the present example, either the hospital or the research institute or both conduct the comparing. The method may further comprise a licensing block indicative of licensing condition for the respective data set. The method may further comprise updating a data block. The distributed ledger may further comprise a licensing block.

Fig. 2 shows a schematic view of an exemplary blockchain 10 of the distributed ledger. The blockchain comprises in the present example a sequence of individual data blocks 11 to 13. Data blocks 11 to 13 represent in the present example the first data sets used to set up the blockchain. Each of the data sets 11 to 13 is provided by a medical institution such as hospital. The step of adding a new data set is depicted with an arrow 15. The new data set 14 is in this example is added to the blockchain 10 after the respective hospitals approved the quality of the new data set as described above. Data block 20 shows an example structure of a data block. The data block comprises a cryptographic hash of the previous data block, a time stamp, an information of the version of the current used blockchain technology, meta data in form of data set details and a summary of total data sets. Data block 20 also comprises a further cryptographic hash relating to its own data set, such that it may always be possible to determine if the data set has been changed.

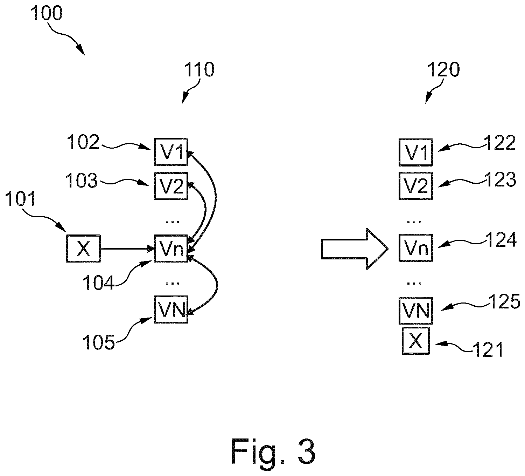

Fig. 3 shows a schematic view 100 of a part of the method, in particular of adding a new data set. A contributor 101 proposes a new data set to a validator 104. The group of validators 110 comprises beside validator 104 further the validators 102, 103 and 105 in the present example. In the present example, the validator 104 broadcasts the proposed new data set to the validators 102, 103 and 105. The group of validators checks the quality of the proposed new data set as described above and adds the new data set as a further data block to the blockchain (i.e. distributed ledger). The group of validators 120 is then enlarged by the contributor 101 of the new added data set. The group of validators comprises then the previous validators 122 to 125 as well as the new validator 121 (i.e. contributor 101).

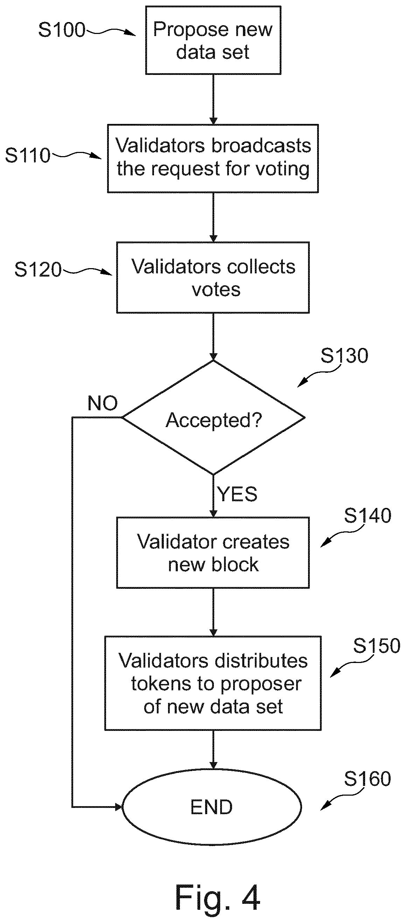

Fig. 4 shows a schematic view of an exemplary embodiment of the method.

Step S100 comprises proposing a new data set. The new data set may be proposed by a white listed Institute in the form of a request. The request may be sent to a single validator in a peer to peer network.

Step S110 comprises broadcasting the request to other validators for checking the quality. The validator that receives the request broadcasts the request to the other validators for checking the quality and for voting whether they want to add the data set to the data pool or not.

Step S120 comprises collecting the votes of the other validators by the single validator.

Step S130 comprises a query whether the collected votes are in favor or against and adding of the proposed new data set. The query may be based on the majority principal or on the unanimity principal. In case, the query relates to a rejection of the proposed new data set the method is continued by step S160. In case the credit relates to a rejection of the proposed new data set the method is continued by step S140.

Step S140 comprises creating a new data block by the single validator. With the new data set is accepted, the single validator creates a new data block with the information of the data set as described above.

Step S150 comprises distributing a token to a proposer of the new data set. This may be linked to the voting where voting yes causes the tokens from that validator to be escrowed to pay for the new data. The new data proposer may be now added to the list of validators.

Step S160 comprises the end of the method.

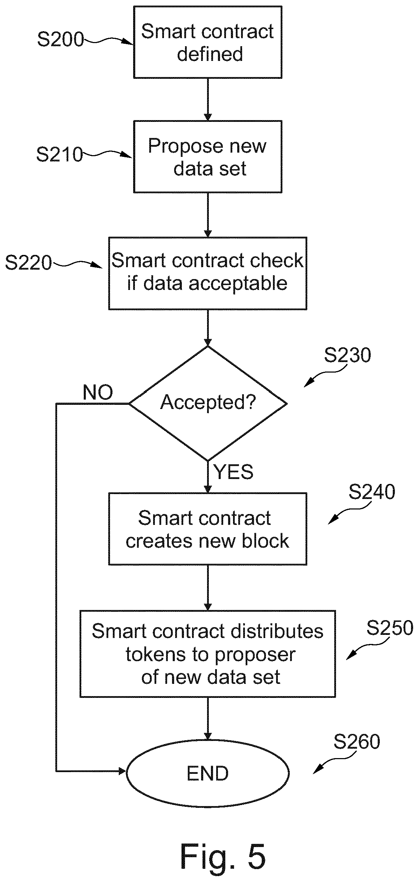

Fig. 5 shows a schematic view of an exemplary embodiment of the method.

Step S200 comprises defining a smart contract. The smart contract may define requirements for a new data set. For example, the smart contract may define the data set requirements (e.g. demographics, size) and the license conditions (e.g. US only) needed to satisfy the contract and the payment for supplying such a dataset. Tokens to pay for the contract may be escrowed beforehand.

Step S210 comprises proposing a new data set. The new data set may be proposed by a white listed institution.

Step S220 comprises executing the smart contract. The smart contract code is executed and checks whether the new data set is acceptable by checking whether the new data set meets the requirements defined by the smart contract.

Step S230 comprises a query whether the data set meets the requirements of the smart contract. In case the new data set is not accepted, the method is continued by step S260. In case that the new data set is accepted, the method is continued by step S240.

Step S240 comprises creating a new data block by the smart contract. The new data block is created based on the proposed new data set.

Step S250 comprises distributing tokens to a proposer of a new data set by the smart contract.

Step S260 comprises the end of the method.

### LIST OF REFERENCE SIGNS

- S10: providing a distributed ledger
- S20: providing an access

- S100: Propose new data set
- S110: Validators broadcasts the request for voting
- S120: Validators collects votes
- S130: Query
- S140: Validator creates new block
- S150: Validators distributes tokens
- S160: End

- S200: Smart contract defined
- S210: Propose new data set
- S220: Smart contract check
- S230: Query
- S240: Smart contract creates new block
- S250: Smart contract distributes token
- S260: END

- 10: blockchain, distributed ledger
- 11: genesis block
- 12, 13: blocks
- 14: new block
- 15: process of adding a new block
- 20: structure of a block
- 101: new participant
- 102, 103, 104, 105: single validator
- 110, 120: group of validators
- 121, 122, 123, 124, 125: group of validators

## Claims

1. Computer implemented method for operating a data pool in a healthcare environment, comprising:
providing a distributed ledger with a plurality of data blocks (S10);
wherein each of the data block comprises at least a cryptographic hash for a respective data set of the data pool and meta data describing the respective data set;
providing an access to the respective data set by an access control method (S20).

2. The method according to claim 1, wherein the respective data set is stored off the distributed ledger.

3. The method according to claim 1 or 2, wherein the access control method comprises key encryption.

4. The method according to any one of the preceding claims, wherein the data block comprise legal information.

5. The method according to any preceding claims, further comprising adding a further data block to the distributed ledger.

6. The method according to claim 5, wherein adding a further data block comprises
comparing the meta data of the further data block with a quality measure and deriving a comparison result; and
adding the further data block based on the comparison result to the distributed ledger.

7. The method according to claim 6, wherein the quality measure comprises one or more of the following: data set size, data set quality, data set novelty, legal status of data set.

8. The method according to any one of the claims 5 to 7, wherein the comparing is carried by a validator, wherein the validator is one of the following: authority, provider of a data set.

9. The method according to any one of the claims 5 to 7, wherein the comparing is carried by a plurality of validator, wherein the validator is one of the following: authority, provider of a data set.

10. The method according to any one of the preceding claims, further comprising adding a licensing block to the distributed, wherein the licensing block comprises at least information on licensing conditions for the respective data set.

11. The method according to any one of the preceding claims, further comprising updating the data block, wherein the updating comprises a replacing of the respective data set with a new data set and replacing the respective data block with a new data block.

12. The method according to any one of the preceding claims, wherein the distributed ledger comprises a summary block, wherein the summary block comprises a summary of all data sets and/or wherein the distributed ledger comprises one or more tokens, wherein the tokens are indicative of a stake on the data sets in the data pool.

13. A device comprising means for carrying out the method according to any one of the claims 1 to 12.

14. A computer program comprising instructions which, when the program is executed by a computer, cause the computer to carry out the method according to any one of the claims 1 to 12.

15. A computer readable medium comprising instructions which, when executed by a computer, cause the computer to carry out the method according to any one of the claims 1 to 12.
